# EUROPEAN PATENT APPLICATION

(11) **EP 2 926 832 A1**
(43) Date of publication of application: **07.10.2015**
(21) Application number: 15161633.1
(22) Date of filing: 30.03.2015
(51) Int. Cl.: A61K 47/12, A61K 9/06, A61K 31/19

(54) **PHARMACEUTICAL FORMULATIONS OF LOXOPROFEN FOR TOPICAL USE**

(30) Priority: 31.03.2014 TR 201403702; 17.04.2014 TR 201404449
(71) Applicant: Sanovel Ilac Sanayi ve Ticaret A.S., 34460 Istanbul (TR)
(72) Inventor: Türkyilmaz, Ali, 34460 Istanbul (TR); Erdem, Yelda, 34460 Istanbul (TR); Terkinli, Alper, 34460 Istanbul (TR); Dedeoglu, Yavuz, 34460 Istanbul (TR); Asik, Cigdem, 34460 Istanbul (TR)
(74) Representative: Sevinç, Erkan

(57) **Abstract**

The present invention relates to pharmaceutical formulation comprising loxoprofen or a pharmaceutically acceptable salt thereof and one or more pharmaceutically acceptable excipients for topical use.

## Description

### Technical Field of the Invention

The present invention relates to pharmaceutical formulation comprising loxoprofen or a pharmaceutically acceptable salt thereof and one or more pharmaceutically acceptable excipients for topical use.

### Background of the Invention

Loxoprofen is a non-steroidal anti-inflammatory drug in the propionic acid derivatives group. It is a prodrug and it is quickly converted to its active trans-alcohol metabolite following oral administration. It is a non-selective cyclooxygenase inhibitor and works by reducing the synthesis of prostaglandins from arachidonic acid. Its chemical name is (RS)-2-{4-[(2-oxocyclopentyl)methyl]phenyl}propanoic acid and its chemical structure is shown in the Formula I.

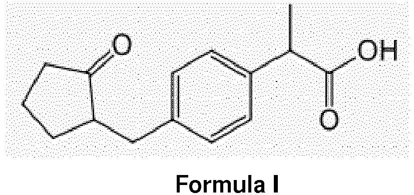

The patent application US4161538 (A) discloses the loxoprofen molecule.

The patent JP2669517 (B2) discloses a solid preparation containing loxoprofen sodium and additives such as microcrystalline cellulose, hydroxypropyl cellulose having low substitution degree, lactose or magnesium stearate, in the form of a tablet, capsule, granule or powder.

The patent EP0947584 (B1) discloses an anti-inflammatory analgesic patch comprising loxoprofen or pharmaceutically acceptable salt thereof, water, crotamiton and a water soluble polymer.

The patent application WO0247661 (A1) discloses pharmaceutical composition for intramuscular injection containing loxoprofen or a pharmaceutically acceptable salt thereof, as an active ingredient.

The patent application JP2010270140 (A) discloses an oral pharmaceutical composition which alleviates gastric mucosa disorders caused by loxoprofen, comprises one or more sugars and sugar alcohols selected from the group consisting of sucrose, maltitol, fructose, xylitol, trehalose, lactose and lactitol; and loxoprofen. A tablet, subtle granule (powder is included), capsule, solution (syrup is included), etc., may be cited as a concrete dosage form of the oral pharmaceutical composition.

The patent EP1806152 (B1) discloses an external preparation containing a pharmacologically active component that is loxoprofen and a lipophilic polyglycerin fatty acid ester.

The patent EP1806151 (B1) discloses an external preparation containing a pharmacologically active component that is loxoprofen and edetic acid salt and aluminum hydroxide.

The patent application EP2116234 (A4) discloses pharmaceutical composition for external application to skin, comprising a drug that is loxoprofen, an auxiliary agent, a pyrrolidone compound comprising N-methyl-2-pyrrolidone and a hydrogenated oil, also, a skin patch comprising a backing, and an adhesive layer laminated on the backing, wherein the adhesive layer comprises a styrene-isoprene-styrene block copolymer, a tackifier, a plasticizer, diclofenac sodium, 1-menthol, N-methyl-2-pyrrolidone, a hydrogenated oil, and at least one of citric acid and lactic acid.

In the state of art, there are several patents and applications which disclose many formulations of loxoprofen or a pharmaceutically acceptable salt thereof, in different dosage forms. In the present invention a novel topical gel formulation of loxoprofen or a pharmaceutically acceptable salt thereof is disclosed.

Formulations for external uses are becoming an increasingly important issue in the area of better patient compliance comparative to the conventional dosage forms for oral administration such as capsules and tablets, which are the most commonly used. In terms of patient compliance, the use of loxoprofen in treating local pains and inflammations may cause a problem especially for those who have gastrointestinal system disorders. Therefore, a need rises in developing locally-administrable topical forms of loxoprofen, in order to avoid the systemic side-effects like gastric mucosa disorders thereof.

The skin absorption rate of the relevant product to be used in topical applications is quite significant. Enhancing the absorption rate both provides ease of application and increases the molecule's efficiency. Particularly in acute disorders, there arises the need of enhancing the absorption rate at the site of administration. In instances during which local pains associated with injuries in sportive events are to be urgently alleviated, it becomes necessary to apply local anesthesia to the relevant site.

Moreover, it is very important to obtain a stable and homogenous topical pharmaceutical composition.

Finally, topical pharmaceutical formulations should have desirable rate of percutaneous penetration, thereby shortening the time period in which the active agents exert their effect and should be stable over the shelf life and show homogeneity. Therefore, absorbtion rate, stability and homogenity are the problems that should be overcome.

### Detailed Description of the Invention

The present invention relates to pharmaceutical formulation comprising loxoprofen or a pharmaceutically acceptable salt thereof and one or more pharmaceutically acceptable excipients for topical use.

According to one embodiment, pharmaceutically acceptable salt of loxoprofen is sodium hydrate.

According to one embodiment, loxoprofen sodium hydrate is present in an amount of 0.5 to 2 % and preferably 0.5 to 1 % by weight of total formulation.

The pharmaceutical formulations according to the present invention may also comprise one or more pharmaceutically acceptable excipient(s). Pharmaceutically acceptable excipients comprise, but are not limited to thickening agents, solvents, penetration enhancer, chelating agents, preservatives, pH balancers or the mixtures thereof.

In a preferred embodiment of the present invention, said thickening agents comprise, but are not limited to carbomer, carbomer copolymers, hydroxypropyl cellulose, hydroxyethyl cellulose, hydroxypropyl methyl cellulose, methyl cellulose, carboxymethyl cellulose, gelatin, aluminum monostearat, dextrin, sodium alginate, pectin, carrageanen, xanthan or the mixtures thereof, preferably, it is carbomer 980.

According to one embodiment, carbomer 980 is present in an amount of 0.1 to 5 % and preferably 0.1 to 3 % by weight of total formulation.

In a preferred embodiment of the present invention, said solvents comprise, but are not limited to polyethylene glycol, ethanol, purified water, ethyl alcohol, glycerin, isopropyl alcohol or the mixtures thereof, preferably, they are polyethylene glycol 400, ethanol and purified water.

In present invention, the topical pharmaceutical formulation comprises polyethelene glycol, ethanol and water as solvents to achieve a favorable solubility of the ingredient of the formulation and desirable homogeneity. Polyethylene glycols (PEGs) are widely used in a variety of pharmaceutical formulations, including parenteral, topical, ophthalmic, oral, and rectal preparations. Polyethylene glycols can be used to enhance the aqueous solubility or dissolution characteristics of poorly soluble compounds. In addition, water is widely used as a solvent in the processing, formulation and manufacture of pharmaceutical products and it is chemically stable in all physical states. Also, ethanol is a good solvent besides its penetration increasing property. Therefore, in this invention, it has surprisingly been found that the use of polyethylene glycol 400, water and ethanol together creates a synergistic effect and leads to better dissolving of the ingredient of the formulation and provides desirable homogeneity.

According to one embodiment, polyethylene glycol 400 is present in an amount of 1 to 20 % and preferably 1 to 10 % by weight of total formulation, ethanol is present in an amount of 1 to 20 % and preferably 1 to 10 % by weight of total formulation.

In a preferred embodiment of the present invention, said penetration enhancer comprise, but are not limited to dimethyl sulfoxide, ethanol, 1,3-didocyl urea, 1,3-difenyl urea, 3-caren, 7-oxabicylo 2,2-heptan, ascaridol, dimetyl isosorbide, dimetyl formamide (DMF), d-Limonen, isopropyl myristat, carveol, carvon, menton, N-metyl-2-pyrolidon, NN-dimetyl toluamide, oleic acid, pinen oxide, puiegon, cyclohexane oxide, cyclopentene oxide, sodyum lauryl sulfate, terpinen-4-ol urea, a-pinen, a-terpineol or mixtures thereof, preferably, they are dimethyl sulfoxide and ethanol.

The skin absorption rate of the relevant product to be used in topical applications is quite significant. Enhancing the absorption rate both provides ease of application and increases the molecule's efficiency. In this invention, dimethyl sulfoxide helps the formulation of the present invention to improve and enhance the penetrating and spreading properties through the skin. It also helps to carry out other components easily and without damaging the membranes into biological system. In present formulation, dimethyl sulfoxide is used with ethanol as penetration enhancers and it has surprisingly been found that the use of dimethyl sulfoxide and ethanol together creates a synergistic effect and provides more desirable rate of percutaneous penetration and spreading through the skin.

According to one embodiment, dimethyl sulfoxide is present in an amount of 1 to 20 % and preferably 1 to 10 % by weight of total formulation.

In a preferred embodiment of the present invention, said chelating agents comprise, but are not limited to citric acid, sodium edetate, diethylenetriaminepentaacetic acid (DTPA), N,N- bis(carboxymethyl) glycine (NTA) or the mixtures thereof, preferably, it is citric acid.

According to one embodiment, citric acid is present in an amount of 0.01 to 2 % and preferably 0.01 to 1 % by weight of total formulation.

In a preferred embodiment of the present invention, said preservatives comprise, but are not limited to methylparaben, propylparaben, sodium and potassium benzoate, imidurea, monothioglicol, phenyl mercuric derivatives, sodium sulfate, sodium meta bisulfate, potassium sorbate or the mixtures thereof, preferably, they are methylparaben and propylparaben.

Topical pharmaceutical formulations should have good stability. In this invention the use of methylparaben and propylparaben provides better stability, helps the formulation to be stable over the shelf life and exhibits high safety when applied to skin

According to one embodiment, methylparaben is present in an amount of 0.01 to 0.5 % and preferably 0.01 to 0.3 % by weight of total formulation, propylparaben is present in an amount of 0.01 to 0.1 % and preferably 0.01 to 0.05 % by weight of total formulation.

According to one embodiment, the pharmaceutical formulation of the present invention comprises triethanolamine as a pH balancer and it is present in an amount of 0.01 to 5 % and preferably 0.01 to 3 % by weight of total formulation.

The formulation according to the present invention may be in the form of topical jel.
In this present invention, the formulation has been designed comprising the following:
a) 0.5 to 1 % by weight of loxoprofen sodium hydrate
b) 0.1 to 3 % by weight of carbomer 980
c) 1 to 10 % by weight of polyethylene glycol 400
d) 0.01 to 3 % by weight of methylparaben
e) 0.01 to 0.05 % by weight of propylparaben
f) 1 to 10 % by weight of dimethyl sulfoxide
g) 0.01 to 1 % by weight of citric acid
h) 0.01 to 3 % by weight of triethanaolamine
i) 1 to 10 % by weight of ethanol
j) purified water (q.s.)

### Example 1 - Topical gel formulation of loxoprofen

| **Ingredients** | **Amount (%)** |
|---|---|
| Loxoprofen sodium hydrate | % 0.5 - % 2.0 |
| Carbomer 980 | % 0.1 - % 5.0 |
| Polyethylene glycol 400 | % 1.0 - % 20.0 |
| Methylparaben | % 0.01 - % 0.5 |
| Propylparaben | % 0.01 - % 0.1 |
| Dimethyl sulfoxide | % 1.0 - % 20.0 |
| Citric acid | % 0.01 - % 2.0 |
| Triethanaolamine | %0.01 - % 5.0 |
| Ethanol | % 1.0 - % 20.0 |
| Purified water | q.s. |

| | |
|---|---|
| *q.s.: quantum sufficient* | |

The pharmaceutical formulation mentioned above is prepared as following:
**a) Preparation of carbomer:** Carbomer 980 is weighed to obtain a 2.2 % (w/w) concentration and dispersed in purified water for four hours, and to swell allowed for a night away from light.
**b) Production of gel:** The thickening agent (carbomer 980) prepared in (a) is mixed with methylpareben and propylparaben which are already dissolved in polyethylenglycol 400. Then, dimethyl sulfoxide is added to the system. And then, citric acid ve loxoprofen sodium hydrate which are dissolved in another beaker is added to the mixture. After the addition of ethanol, triethanolamine is added and continued to mixing for a determined time period for obtaining a homogenous product.

## Claims

1. A topical pharmaceutical formulation comprising loxoprofen or a pharmaceutically acceptable salt thereof and one or more pharmaceutically acceptable excipient.

2. The topical pharmaceutical formulation according to claim 1, wherein pharmaceutically acceptable salt of loxoprofen is sodium hydrate.

3. The topical pharmaceutical formulation according to claim 1 or 2, wherein loxoprofen sodium hydrate is present in an amount of 0.5 to 2 % and preferably 0.5 to 1 % by weight of total formulation.

4. The topical pharmaceutical formulation according to claim 1 or 2 or 3, wherein one or more pharmaceutically acceptable excipient is selected from the group comprising thickening agents, solvents, penetration enhancer, chelating agents, preservatives, pH balancers or the mixtures thereof.

5. The topical pharmaceutical formulation according to claim 4, wherein the thickening agent is selected from the group comprising carbomer, carbomer copolymers, hydroxypropyl cellulose, hydroxyethyl cellulose, hydroxypropyl methyl cellulose, methyl cellulose, carboxymethyl cellulose, gelatin, aluminum monostearat, dextrin, sodium alginate, pectin, carrageanen, xanthan or the mixtures thereof, preferably, it is carbomer 980.

6. The topical pharmaceutical formulation according to claim 5, wherein carbomer 980 is present in an amount of 0.1 to 5 % and preferably 0.1 to 3 % by weight of total formulation.

7. The topical pharmaceutical formulation according to claim 4, wherein the solvent is selected from the group comprising polyethylene glycol, ethanol, purified water, ethyl alcohol, glycerin, isopropyl alcohol or the mixtures thereof, preferably, they are polyethylene glycol 400, ethanol and purified water.

8. The topical pharmaceutical formulation according to claim 7, wherein polyethylene glycol 400 is present in an amount of 1 to 20 % and preferably 1 to 10 % by weight of total formulation and ethanol is present in an amount of 1 to 20 % and preferably 1 to 10 % by weight of total formulation.

9. The topical pharmaceutical formulation according to claim 4, wherein the penetration enhancer is selected from the group comprising dimethyl sulfoxide, ethanol, 1,3-didocyl urea, 1,3-difenyl urea, 3-caren, 7-oxabicylo 2,2-heptan, ascaridol, dimetyl isosorbide, dimetyl formamide (DMF), d-Limonen, isopropyl myristat, carveol, carvon, menton, N-metyl-2-pyrolidon, NN-dimetyl toluamide, oleic acid, pinen oxide, puiegon, cyclohexane oxide, cyclopentene oxide, sodyum lauryl sulfate, terpinen-4-ol urea, a-pinen, a-terpineol or mixtures thereof, preferably, they are dimethyl sulfoxide and ethanol.

10. The topical pharmaceutical formulation according to claim 9, wherein dimethyl sulfoxide is present in an amount of 1 to 20 % and preferably 1 to 10 % by weight of total formulation.

11. The topical pharmaceutical formulation according to claim 4, wherein the chelating agent is selected from the group comprising citric acid, sodium edetate, diethylenetriaminepentaacetic acid (DTPA), N,N- bis(carboxymethyl) glycine (NTA) or the mixtures thereof, preferably, it is citric acid.

12. The topical pharmaceutical formulation according to claim 11, wherein citric acid is present in an amount of 0.01 to 2 % and preferably 0.01 to 1 % by weight of total formulation.

13. The topical pharmaceutical formulation according to claim 4, wherein the preservative is selected from the group comprising methylparaben, propylparaben, sodium and potassium benzoate, imidurea, monothioglicol, phenyl mercuric derivatives, sodium sulfate, sodium meta bisulfate, potassium sorbate or the mixtures thereof, preferably, they are methylparaben and propylparaben.

14. The topical pharmaceutical formulation according to claim 13, wherein methylparaben is present in an amount of 0.01 to 0.5 % and preferably 0.01 to 0.3 % by weight of total formulation, propylparaben is present in an amount of 0.01 to 0.1 % and preferably 0.01 to 0.05 % by weight of total formulation.

15. The topical pharmaceutical formulation according to claim 4, wherein pH balancer is triethanolamine and it is present in an amount of 0.01 to 5 % and preferably 0.01 to 3 % by weight of total formulation.

16. The topical pharmaceutical formulation according to any of the preceding claims comprising;
a) 0.5 to 1 % by weight of loxoprofen sodium hydrate
b) 0.1 to 3 % by weight of carbomer 980
c) 1 to 10 % by weight of polyethylene glycol 400
d) 0.01 to 3 % by weight of methylparaben
e) 0.01 to 0.05 % by weight of propylparaben
f) 1 to 10 % by weight of dimethyl sulfoxide
g) 0.01 to 1 % by weight of citric acid
h) 0.01 to 3 % by weight of triethanaolamine
i) 1 to 10 % by weight of ethanol
j) purified water (q.s.)
